(19) **Europäisches Patentamt**
**European Patent Office**
**Office européen des brevets**

(11) **EP 2 060 550 A1**

(12) **EUROPEAN PATENT APPLICATION**

(43) Date of publication:
**20.05.2009 Bulletin 2009/21**

(51) Int Cl.:
**C07C 1/20** *(2006.01)*　　**C07C 9/16** *(2006.01)*
**C07C 11/02** *(2006.01)*　　**B01J 29/06** *(2006.01)*

(21) Application number: **07254486.9**

(22) Date of filing: **16.11.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **BP p.l.c.**
**London SW1Y 4PD (GB)**

(72) Inventor: **The designation of the inventor has not yet been filed**

(74) Representative: **Oldroyd, Richard Duncan et al**
**BP International Ltd**
**Global Patents & Technology Law (Central)**
**Chertsey Road**
**Sunbury-on-Thames, Middx TW16 7LN (GB)**

(54) **Process for producing triptane**

(57)　　A process for producing triptane and/or triptene from methanol and/or one or more derivatives thereof, and optionally one or more further alcohols and/or derivatives thereof at a temperature in the range of from 150 to 400°C, in the presence of a zeolite catalyst having Brønsted acidity, in which all the carbon atoms in the triptane and/or triptene are derived from the methanol and/or one or more derivatives thereof and the optional further alcohols and/or derivatives thereof, the zeolite catalyst being selected from:
i. zeolites having frameworks comprising silicon and aluminium atoms at a Si:Al mole ratio of greater than 2, which zeolites also have a channel structure comprising a ring size of 12 or more non-oxygen atoms in 2 or 3 dimensions; and
ii. zeolites having frameworks comprising silicon and aluminium atoms, and which have a framework ring comprising 12 or more non-oxygen-atoms accessible on the external surface of the zeolite and a pore structure in which all of the channels have a ring-size of less than 12 non-oxygen atoms.

There is also described a process for producing triptane and/or triptene wherein the zeolite catalyst is zeolite X, the reaction composition additionally comprises a $C_3$ alcohol and/or one or more derivatives thereof, in which the mole ratio of $C_3$ alcohol : methanol present in the reaction composition, or derivable from the one or more derivatives thereof present in the reaction composition, is greater than 0.23 : 10, and the temperature of the process is in the range of greater than 200° to 400°C.

EP 2 060 550 A1

**Description**

This invention relates to the production of triptane, more specifically to the production of triptane from a feedstock comprising one or more oxygen-containing carbon compounds.

In gasoline formulations, branched hydrocarbons are generally favoured over linear hydrocarbons as they result in a fuel with a higher octane number. An example of a branched chain hydrocarbon desirable in gasoline formulations due to its high octane number is triptane, which can be used in unleaded aviation gasoline and unleaded motor gasoline, as described for example in WO 98/22556 and WO 99/49003.

Branched chain hydrocarbons may be synthesised by a number of routes. In particular, mixtures of branched chain hydrocarbons may be formed by homologation of methanol and/or dimethyl ether in the presence of a zinc halide catalyst, as described, for example in GB 1,547,955, US 2,492,984, US 3,969,427, US 4,059,646, US 4,059,647, US 4,249,031 and WO 02/70440.

For example, US 4,249,031, describes a process for the preparation of a hydrocarbon mixture by contacting one or more oxygen-containing organic compounds, such as methanol, with one or more zinc halides, while US 4,059,647 describes a process for the production of triptane (2,2,3-trimethylbutane) comprising contacting methanol, dimethyl ether or mixtures thereof with zinc iodide.

Olefins can also be reacted with alcohols, in particular methanol, to produce hydrocarbons. For example, US 4,151,214 describes use of zinc bromide or iodide catalysts in the conversion of methanol or dimethyl ether with an olefin.

However, a common problem associated with the use of halide-containing catalysts is that the halides can contribute to corrosion problems in plant equipment. Furthermore, halide from the catalyst can contaminate the triptane product, and hence needs to be removed before it can be used as a fuel.

Zeolites have been reported as useful in the production of gasoline components from methanol, as described for example in US 3,894,102 and US 3,894,107. However, the products are mainly aromatic in nature, and nothing is disclosed or taught about whether triptane is or could be created.

There remains a need for an alternative process for producing triptane.

According to a first aspect of the present invention, there is provided a process for the production of triptane and/or triptene from methanol and/or one or more derivatives thereof and optionally one or more further alcohols and/or derivatives thereof, which process comprises contacting a reaction composition comprising methanol and/or one or more derivatives thereof, and optionally one or more further alcohols and/or derivatives thereof, with a zeolite catalyst having Brønsted acidity to produce triptane and/or triptene at a combined yield of greater than 0.05% at a temperature in the range of from 150 to 400°C, in which all the carbon atoms of the triptane and/or triptene are derived from the methanol and/or one or more derivatives thereof and optional further alcohols and/or derivatives thereof, characterised in that the catalyst is selected from one or more of:

    i. zeolites having frameworks comprising silicon and aluminium atoms at a Si:Al mole ratio of greater than 2, which zeolites also have a channel structure comprising a ring size of 12 or more non-oxygen atoms in 2 or 3 dimensions; and
    ii. zeolites having frameworks comprising silicon and aluminium atoms, and which have a framework ring comprising 12 or more non-oxygen-atoms accessible on the external surface of the zeolite and a pore structure in which all of the channels have a ring-size of less than 12 non-oxygen atoms.

According to a second aspect of the present invention, the zeolite catalyst is zeolite X, the reaction composition additionally comprises a $C_3$ alcohol and/or one or more derivatives thereof, in which the mole ratio of $C_3$ alcohol : methanol present in the reaction composition, or derivable from the one or more derivatives thereof present in the reaction composition, is greater than 0.23 : 10, and the temperature of the process is in the range of greater than 200°C to 400°C.

The inventors have found that certain crystalline aluminosilicate zeolites can act as catalysts for the production of triptane (2,2,3-trimethylbutane) and/or triptene (2,3,3-trimethylbut-1-ene), henceforth collectively referred to as triptyl compounds, from a reaction composition comprising methanol and/or one or more derivatives thereof. All the carbon atoms of the triptyl compounds are derived from the methanol and/or derivatives thereof, and also further alcohols and/or derivatives thereof that may also be present in the reaction composition. The zeolites of the present invention all comprise framework silicon and aluminium atoms, have at least some Brønsted acid character, and also comprise either a pore structure having a pore size of 12 non-oxygen atoms or more in 2 or 3 dimensions, or alternatively have a framework feature comprising a ring of 12 or more non-oxygen atoms on the surface of the zeolite structure, the pore structure having a pore size of less than 12 non-oxygen atoms in all dimensions. As the crystalline aluminosilicate catalysts of the present invention do not comprise halide ions, then problems associated with halide contamination of the product stream and halide-induced corrosion in process equipment can be avoided.

Crystalline zeolites typically comprise an ordered inorganic oxide framework structure having a regular array of pores, often with enlarged cages where two or more pores intersect. Most commonly, the framework principally comprises aluminium and silicon atoms bound by oxygen. However, often zeolites can comprise other framework heteroatoms, for example phosphorus, gallium, titanium, germanium, vanadium, iron and cobalt, and these can also be suitable for use in the present invention. When framework heteroatoms are present, they are typically at levels of up to 10 mol%.

The ring size is defined by the number of non-oxygen atoms present in the ring. For example, in the case of an aluminosilicate zeolite having a 12-membered ring, the ring comprises a total of 12 silicon and aluminium atoms. If the zeolite is a silico-aluminophosphate, then the ring comprises a total of 12 silicon, aluminium and phosphorus atoms. Non-framework atoms, for example charge balancing ions such as protons, alkalimetal ions or other positively charged cations which are not incorporated into the zeolite framework, are not considered to be part of the 12-membered ring.

There are two types of zeolite structure that are able to provide a combination of high triptyl yields and also high triptyl fractions in the $C_7$ hydrocarbons. These are henceforth labelled zeolite types (i) and (ii) for convenience.

Type (i) zeolites comprise a 2 or 3-dimensional network of pores, the ring size of the pores being 12 or more non-oxygen framework atoms in at least 2 dimensions. Zeolites in this category include zeolites of the FAU, BEA or EMT structures. Full details of zeolite structures can be found in the Atlas of Zeolite Structure Types, available from the International Zeolite Association.

In one embodiment of the invention, the zeolite has the faujasite (FAU) structure, such as zeolite Y, which comprises channels with 12-membered ring channel openings, with a diameter of around 7.4 Å. The channel structure is 3-dimensional, and has cages with a diameter of around 12.7 Å. Typical framework Si/Al molar ratios of zeolite X are up to 2. For zeolite Y, the ratio is typically above 2.

Zeolite USY, short for "Ultra-Stable" Y, or zeolite VUSY, short for "Very Ultra-Stable" Y, are produced when zeolite Y is subjected to a dealumination treatment, typically using high temperature steam treatment. This results in aluminium being extracted from the framework, resulting in so called extra-framework alumina particles and leaving a disrupted zeolite framework structure. At high levels of dealumination, for example in the case of VUSY, the zeolite is not so active towards triptyl formation, and typically only exhibiting significant triptyl yields and fractions at temperatures above 275°C, for example at 350°C or above. This is possibly a result of the number and density of acid sites in the framework being reduced to too great an extent for reaction to occur. However, it is believed that a lesser degree of dealumination may be advantageous because a disrupted framework can allow improved access to a greater number of acidic sites of the zeolite. Therefore, in dealuminated zeolites, a balance must be made between improving access to the acidic sites of the zeolite, and maintaining sufficient framework acid site density to enable the triptyl-forming reaction to occur.

Zeolite X has the FAU structure, but has a Si/Al molar ratio of 2 or less. It has been found to be active in forming triptane, but under different conditions than zeolite Y, for example, which will be described in more detail below.

Another zeolite structure having 12-membered ring channel openings is the BEA structure, as exhibited by zeolite Beta, which also has a 3-dimensional channel structure. Si/Al molar ratios of aluminosilicate zeolite Beta are typically from 5 to 100, as described in US 3,308,069.

Yet another zeolite structure having 12-membered rings and a 3-dimensional pore structure is the EMT structure, as exhibited for example by zeolite ZSM-3, as described in US 3,415,736, and by zeolite ZSM-20 as described in US 3,972,983.

Zeolites of structure type (ii) have pores with a ring size of 10 or less in all dimensions. However, the framework structure comprises features having 12 or more-membered rings available at the external surface of the zeolite.

An example of such a structure is the MWW structure, which has a two-dimensional pore structure, in which all the pores in the network have a 10-membered ring at their narrowest portion. However, the channels intersect at cylindrical cages formed of 12-membered rings, the cage dimensions being about 7.1Å diameter across the short axis and about 18.2Å in length. Thus, although access to the 12-membered ring cages is restricted by the 10-membered ring openings, the external surfaces of the crystals comprise opened cages, thus making the 12-membered rings accessible to reactants. MCM-22, described in US 4,992,615, is an example of an aluminosilicate zeolite with this structure, typically having a Si/Al ratio of 5 or more. In MCM-22, the cages appear as cups or pockets on the crystal surface, having a diameter of about 7.1Å and a depth of about 7.0Å.

Other examples of zeolites suitable for use in the present invention are delaminated zeolites. ITQ-2 is an example of a delaminated zeolite. The ITQ-2 framework is based on that of the layered MCM-22 (MWW) structure, of the structure type (ii), except that condensation of the MCM-22 layers at the framework cages is disrupted during the synthesis, resulting in a disordered layered structure and an increased proportion of 12-membered ring cages open and accessible to reactants at the external crystal surface. The synthesis and structure of ITQ-2 is described in WO 01/21562.

For the zeolite to have Brønsted acidity, the overall charge on the framework must be negative, such that positive ions, in particular protons, are required to balance the charge. The framework of aluminosilicate zeolites is negatively charged, which can be balanced by protons to produce Brønsted acid sites. This type of Brønsted acidity is represented below in equation I.

$$[(SiO_2)_x(SiAlO_2)_y]^{y-} \cdot yH^+ \qquad\qquad \text{Equation I}$$

In another embodiment, the framework negative charge can be balanced by another cation, for example a transition metal or lanthanide ion, which itself can possess Brønsted acid character, for example by deprotonation of coordinated water molecules. This type of Brønsted acidity is represented below in equation II.

$$[(SiO_2)_x(SiAlO_2)_y]^{y-} \cdot (y/z)M^{z+} \cdot (H_2O)_a \cdot (OH^-) \cdot H^+ \qquad \text{Equation II}$$

Typically, the negative charges of the framework are counter-balanced by protons. The zeolite should have a sufficient number of acid sites to enable the acid-catalysed reaction of methanol and/or derivatives thereof to form triptyl compounds to proceed. This can be conveniently expressed in terms of the framework silicon to aluminium mole ratio, which is typically 100 or less, for example 80 or less, such as 50 or less.

Optionally, the negative charge of the zeolite framework can be partially counter-balanced by cations other than protons, for example ammonium ions or one or more alkalimetal ions, for example one or more of sodium, potassium, rubidium and caesium. Additionally, or alternatively, one or more alkaline-earth ions can be present, for example one or more of magnesium, calcium, strontium and barium. Additionally or alternatively, one or more p-block metal metals, transition metals and lanthanides can be present, for example one or more of lanthanum, cerium, silver, bismuth, copper, cobalt, indium, zinc, rhodium, platinum and palladium.

The number of acid sites in a zeolite can also be modified by treating the zeolite with organic silicon compounds or silicon compounds comprising halides, for example alkoxy silanes, alkyl silanes, silicon halides, or alkyl silicon halides. Specific examples include tetraethoxysilane, tetramethylsilane, silicon tetrachloride, and dimethyl dichloro silane. These react at proton sites, and hence by controlling the level of treatment a controlled quantity of acid sites can be blocked. Treated zeolites are typically treated by calcination in air at a temperature in the range of 400 to 600°C after treatment with the silicon compound.

The loading of the non-framework metals can be conveniently expressed in terms of a molar ratio compared to the element or elements of the zeolite responsible for imparting negative change to the zeolite framework. Such elements are henceforth referred to as T-elements for brevity. For example, in aluminosilicate zeolites, aluminium is the T-element. In gallo-aluminosilicate zeolites, both gallium and aluminium are T-elements. Thus, the loading can be expressed in terms of the total quantity of non-framework metal compared to the total quantity of T-element(s) in the zeolite.

On this basis, the loadings of non-framework metal, where present, are typically 1mol% or more compared to the total T-element content of the zeolite. More preferably, the loadings are in the range of from 1 to 10mol% of the total T-element content of the zeolite. Of the metal-loaded zeolites, it has been found that copper, silver, cobalt and indium appear to give better triptyl yields than the other metals.

For metal-loaded zeolite X, it appears as though the loading has to be greater than 1% of the T-element content of the zeolite for triptyl activity to be observed. In addition, only certain metals produce zeolite X catalysts active for triptyl formation. Without being bound by any theory, it is possible that some of the metals are able to stabilise the zeolite X framework from degradation through dealumination, and that this requires a loading of above 1mol% of the T-element content of the zeolite in order to be effective. In the case of zinc, a loading of greater than 2.5mol% is required.

The reaction that is catalysed is the production of triply compounds, i.e. triptane, triptene or mixtures thereof, from a reaction composition comprising methanol and/or one or more derivatives thereof, and optionally one or more further alcohols and/or derivatives thereof. The carbon atoms in the triptane all derive from the methanol and/or derivatives thereof, and further alcohols and/or derivatives thereof where present. A derivative of methanol is a compound that releases methanol through a hydrolysis reaction, examples of which include methyl esters, methyl ethers and methyl carbonates. Preferred examples of methanol derivatives are dimethyl ether and dimethyl carbonate. In a preferred embodiment, the reaction composition comprises methanol and/or dimethyl ether.

Optionally, one or more further alcohols and/or derivatives thereof are also present in the reaction composition. Typically, when present, they are selected from $C_2$ to $C_6$ alcohols and/or derivatives thereof, an alcohol derivative being a compound which liberates the alcohol on hydrolysis, such as esters or ethers. Typically, the further oxygen-containing compounds are selected from $C_2$ to $C_4$ alcohols and/or derivatives thereof, for example from one or more of ethanol, iso-propanol, n-propanol, iso-butanol, sec-butanol, n-butanol, diethyl ether, di n-propyl ether, and di-n-butyl ether.

One or more of the methanol and/or derivatives thereof, or the optional alcohols and/or derivatives thereof can be biologically derived. For example ethanol and butanol can be produced from the fermentation of biomass. This is advantageous, as the use of biologically-derived feedstocks can result in the triptane having a lower impact on atmos-

pheric $CO_2$-emissions when combusted, because biomass ultimately derives from atmospheric $CO_2$.

It has been found that $C_3$ alcohols, particularly n-propanol, are particularly advantageous components of the reaction composition. Preferably, the oxygen-containing $C_3$ carbon compound is a $C_3$ alcohol, more preferably n-propanol. When present in the reaction composition, the mole ratio of $C_3$ alcohol : methanol present in the reaction composition, or derivable from the one or more derivatives thereof present in the reaction composition, is suitably up to 2 : 1. It is preferably 0.1 : 10 or more, for example 0.5 : 10 or more, such as 1 : 10 or more.

By alcohols derivable from an alcohol derivative is meant the number of alcohol molecules produced in the event of hydrolysis of an alcohol derivative. For example, in the case of dimethyl ether (DME), two methanol molecules would result from hydrolysis, so each dimethyl ether molecule would contribute two methanol molecules. Similarly, a molecule of dimethyl carbonate (DMC) for example would also produce two molecules of methanol on hydrolysis. Thus, a n-propanol : DME or DMC mole ratio of 0.5 : 10 provides an n-propanol : methanol mole ratio of 0.5 : 20.

For zeolite X, a $C_3$ alcohol and/or one or more derivatives thereof are required for triptyl activity to be observed. Preferably, the $C_3$ compound is preferably n-propanol. The mole ratio of $C_3$ alcohol : methanol present in the reaction composition, or derivable from the one or more derivatives thereof present in the reaction composition, is greater than 0.23 : 10.

Without being bound by any theory, it is thought that the advantageous features of the catalysts used in the process of the present invention can be attributed to the need for a relatively large pocket, cage or channel which can accommodate the triptyl molecules when they are formed, while reducing any restriction that may prevent the triptyl molecules diffusing away from the catalyst. Thus, in the case of MCM-22 and ITQ-2 for example, the surface 12-membered ring pockets allow the triptyl molecule to form, but the small channels having 10-membered ring restrictions prevents their diffusion into the zeolite framework. In the case of zeolite Y or Beta, for example, although the 12-membered ring channels allow access to the interior of the zeolite structure, diffusion is not inhibited as the channel structure is interconnecting in 3-dimensions, allowing a facile route out of the catalyst interior.

In the present invention, zeolites MCM-22, Y and Beta have been shown to be particularly effective towards triptyl formation. Of these, MCM-22 and Y are preferred as they show higher activity over a wider range of reaction conditions and a wider selection of feedstocks.

In a typical process, a feedstock comprising methanol and/or one or more derivatives thereof, and optionally further alcohols and/or derivatives thereof are fed to a reactor in which the zeolite catalyst resides. A product stream is produced, comprising unreacted reactants, triptyl compounds and other by-products, which is removed from the reactor. An inert diluent can also optionally be fed to the reactor. A typical diluent is selected from nitrogen, argon, or an alkane such as methane or ethane. The inert diluent is stable and unreactive under the conditions employed in the reaction. Where the feedstock comprises more than one compound, they may be pre-mixed before being fed to the reactor, or alternatively they may be fed separately to the reactor.

The triptyl-containing product stream can be treated so as to separate and/or purify triptyl compounds from the unreacted reactants and by-products. Optionally, unreacted reactants and one or more by-products can be recycled to the reactor. Separation and purification typically utilises one or more apparatus selected from flash separation vessels, distillation columns, decantation vessels and solid adsorbent beds.

It may not be necessary to completely isolate and purify the triptyl compounds. For example, in one embodiment, the product stream is treated so that triptyl compounds are present at a concentration sufficient for them to be blended with a gasoline fuel.

The reaction composition can additionally comprise hydrogen, optionally diluted with a diluent. The presence of hydrogen can improve triptyl yields. Optionally, the zeolite can comprise metals active as hydrogenation catalysts, for example Rh, Pt or Pd.

Without being bound by any theory, it is believed that the catalysts of the present invention proceed by first causing the dehydration of alcohols to ethers and/or olefins, for example methanol to dimethyl ether, or propanol to propene and/or dipropyl ether. Such reactions are known to be catalysed by acids. The next step is the coupling or condensation of the dehydrated intermediates, for example the ethers and/or olefins, to larger hydrocarbon molecules, which results in the formation of mixtures of hydrocarbons. The triptyl compounds, being highly branched in nature, are quite bulky. Thus, in the case of zeolites, it is thought that a framework ring of 12 or more non-oxygen atoms is necessary so that the ring is of sufficient size to accommodate the relevant reactive intermediates needed for triptyl formation. The ring is preferably a 12-membered ring, as zeolites with 12-membered rings are typically more stable than those with larger ring sizes.

Typically, the reactions are conducted so that reactants are in the gas-phase, which usually require temperatures in the range of from 100 to 400°C, and preferably in the range of from 200 to 350°C.

Where the catalyst is zeolite X, the temperature is above 200°C and up to 400°C, more preferably the temperature is above 275°C, for example in the range of from 300 to 400°C.

The total gas hourly space velocity (GHSV) of the reactants and optional diluent over the catalyst is suitably up to 5 000 $h^{-1}$, preferably less than 3000 $h^{-1}$. The GHSV is also preferably above 500 $h^{-1}$, for example in the range of from 1000 to less than 3000 h$^{-1}$, for example in the range of from 1000 to 2000 h$^{-1}$.

The GHSV is given in units of mL gas, corrected to 0°C and 1 atm pressure, per mL catalyst per hour. The reaction pressure can be in the range of 1 bara (0.1 MPa) up to 100 bara (10 MPa). Higher pressures tend to suppress triptyl production, hence the pressure is preferably below 30 bara (3 MPa), for example less than 20 bara (2 MPa).

In one embodiment of the invention, a mixture of carbon monoxide and hydrogen can be used to produce methanol and/or one or more derivatives thereof, and optionally further alcohols and/or derivatives thereof, in-situ within the reactor. This can be achieved by adapting the catalyst to comprise one or more additional components that are active for the production of methanol and/or derivatives thereof, and optionally additional alcohols and/or derivatives thereof from the carbon monoxide and hydrogen (henceforth referred to as an alcohols synthesis catalyst). In this way, the alcohols-containing reaction composition from which the triptyl compounds are produced can be generated directly from feedstocks such as syngas, which can be derived from natural gas, coal or biomass for example.

In this embodiment, the catalyst is suitably layered, such that the carbon monoxide and hydrogen-containing feedstock contacts the alcohols synthesis catalyst first, the so-formed methanol and/or one or more derivatives thereof and optionally further alcohols and/or derivatives thereof subsequently contact a layer of the zeolite catalyst of the present invention to form triptyl compounds.

In one embodiment of the invention, the alcohols synthesis catalyst is predominantly active for the formation of methanol and/or derivatives thereof, an example being a copper /zinc oxide/alumina (Cu/ZnO/Al$_2$O$_3$) catalyst.

In another embodiment, the catalyst is suitable for the production of mixed alcohols, such as a mixture of C$_1$ to C$_4$ alcohols and/or derivatives thereof, for example a cobalt-molydenum-sulphide catalyst (CoMoS).

The alcohols synthesis catalyst can, in a further embodiment, be mixed with an acidic catalyst, for example high surface area alumina, in order to increase the selectivity of the reaction towards ethers.

In an alternative embodiment, the process comprises two reactors, a first reactor having the alcohols synthesis catalyst, and a second reactor having the zeolite catalyst for triptyl formation. The carbon monoxide and hydrogen are fed to the first reactor to produce a product composition comprising methanol and/or one or more derivatives thereof, and optionally further alcohols and/or derivatives thereof, are produced, which then form at least part of the reaction composition for the triptyl-forming reaction. Optionally, before being fed to the second reactor, the product composition from the first reactor is treated to remove unwanted components, such as unreacted carbon monoxide and/or hydrogen, achieved, for example, through flash separation, and/or to remove by-products such as water, which can be achieved for example by distillation or by use of a selective molecular sieve absorbent. As optimum operating conditions of the alcohols forming and triptyl forming reactions are likely to be different, this separate reactor embodiment offers an advantage in enabling different operating conditions to be used for the two separate reactions.

Mixtures of carbon monoxide and hydrogen, where used, preferably have a H$_2$:CO mole ratio of 1:1 or above, for example in the range of 1:1 to 10:1, and more preferably in the range of from 1:1 to 6:1. Optionally, carbon dioxide can also be a constituent of the feedstock. Where present, the H$_2$ CO$_x$ (i.e. CO + CO$_2$) mole ratio is suitably in the range of from 0.5:1 to 20:1, for example in the range of from 1:1 to 15:1, or 1:1 to 10:1.

Where the process comprises a mixed catalyst, and the alcohols forming and triptyl forming reactions occur within the same reactor, the temperature is preferably less than 310°C and is also preferably at least 240°C. More preferably, the temperature is in the range of from 250 to 300°C. The pressure is typically at least 10 bara (1 MPa) and typically at most 200 bara (20 MPa), for example up to 100 bara (10 MPa). Preferably, the pressure is at least 30 bara (3 MPa), and more preferably in the range of from 40 to 60 bara (4 to 6 MPa).

Where the alcohols-forming reaction and catalyst are in a separate reactor to the triptyl-forming reaction, the alcohols-production reaction typically operates at a temperature in the range of from 150 to 400°C, for example in the range of from 240 to 300°C, and a pressure in the range of from 10 to 150 bara (1 to 15 MPa), for example in the range of from 50 to 150 bara (5 to 15 MPa).

The alcohol and/or ether-containing product stream (including methanol and/or dimethyl ether) does not necessarily need to be treated to remove higher alcohols and/or ethers, as the higher alcohols and/or ethers can be useful components of the reaction composition for triptane formation.

Optionally, the product stream from the triptyl-forming reaction is contacted with hydrogen in a separate reactor, in the presence of a hydrogenation catalyst such as a supported ruthenium, palladium or platinum catalyst, the support being for example carbon, alumina or silica. This converts olefins to the corresponding alkanes.

The zeolite catalysts of the present invention can enable greater than equilibrium yields of triptyl compounds to be produced, when expressed as a fraction of the C$_7$ hydrocarbons produced in the reaction. From thermodynamic considerations, at temperatures between 200 and 350°C, the fraction of triptane compared to all non-cyclic C$_7$ alkanes is expected to be in the range of 2 to 3% on a molar basis. In the present invention, the zeolite catalysts of the present invention enables triptyl fractions compared to all C$_7$ compounds of greater than 5% on a molar basis to be achieved. In addition, using the zeolite catalysts in the process of the present invention enables triptyl yields of greater than 0.05% to be achieved, based on the total carbon in the reaction composition (excluding any carbon associated with inert diluent).

Experimentally, the triptyl fraction is determined by calculating the percentage yield of triptyl compared to all products appearing in a GC trace at a retention time exceeding that of n-hexane, up to and including n-heptane, when using a GC column that separates components on the basis of boiling point, such as a DB-1 or CP-Sil column.

The invention will now be illustrated by the following non-limiting examples, and with reference to the Figures in which;

Figure 1 is a graph showing the yield of triptyl compounds for a series of zeolites in their protonated form for reaction of a nitrogen-diluted methanol/propanol feedstock.

Figure 2 is a graph comparing catalytic activity over time for three different protonated zeolites under the same conditions.

Figure 3 is a graph showing triptyl fractions obtained from various zeolites.

Figure 4 is a graph comparing catalytic activity for MCM-22 at different GHSVs.

Figure 5 is a graph comparing the triptyl fractions for various zeolites using a feedstock of nitrogen-diluted dimethylether and n-propanol.

Figure 6 is a graph comparing the triptyl fractions for various zeolites using a feedstock of nitrogen-diluted methanol.

Figure 7 is a GC trace of the gaseous portion of a product stream resulting from a feedstock of nitrogen-diluted methanol and n-propanol being fed over zeolite ZSM-5.

Figure 8 is a GC trace of the gaseous portion of a product stream resulting from a feedstock of nitrogen-diluted methanol and n-propanol being fed over zeolite MCM-22.

Catalytic experiments were conducted using a multi-reactor block comprising 16 single pass tube reactors each of 2mm internal diameter. Catalyst particles of 50 to 200 micrometers in diameter were loaded into the reactors. Each reactor had a dedicated and separate supply of reactants and inert diluent and separate liquid collection vessels for collection of high-boiling components that could cause fouling of the gas analysis equipment. Gas-phase material coming out of the reactors were fed to a common GC apparatus for analysis.

Zeolites were either purchased from commercial sources, or were prepared using literature methods where not commercially available. They were converted to the ammonium-ion form by a triple ion-exchange treatment with 0.2M ammonium nitrate solution, the material being decanted and water-washed between each ion exchange treatment. The resulting material was left to dry at 50°C, and calcined in air at 400°C. Before use, the ammonium-exchanged material was heated under nitrogen at 400°C to convert to the acid form. Reported activities relate to the first 400 minutes of the reaction, unless otherwise stated.

Metal-loaded zeolites were prepared by incipient wetness impregnation, by evaporation a suspension of the zeolite in an aqueous solution of relevant soluble metal salts to dryness at a temperature of 50-60°C. The material was then dried at 110°C for 1 hour, and calcined in air overnight at 500°C.

In all experiments, triptyl formation is defined as a triptyl yield of greater than 0.05%, versus all carbon in the reaction composition. Triptyl yields (where observed) were typically up to 3%. The main other hydrocarbon products observed were non-triptyl hydrocarbons having 4 to 8 carbon atoms, with some oligomeric hydrocarbons with more than 8 carbon atoms also being apparent, in addition to some methylated aromatics. Dimethyl ether, resulting from methanol dehydration, was also observed, as was water resulting from dehydration of any alcohols present in the reaction composition.

Unless otherwise specified, product analysis was carried out by gas chromatography using an analyser fitted a DB-1 column, a flame ionisation detector, with hydrogen being used as the carrier gas.

Where triptyl fractions are quoted, these relate to the percentage of triptyl compounds compared to products appearing in the GC trace (based on a DB-1 or CP-Sil column) at retention times greater than n-hexane, up to and including n-heptane. This closely relates to the yield of triptyl compounds as a percentage of $C_7$ hydrocarbons.

With the exception of zeolite X, all zeolites had a silicon : aluminium mole ratio within the range of greater than 2 and up to 50. MCM-22 samples had an Si:Al mole ratio of 25. Zeolite X had an Si:Al mole ratio of 1.2.

Experiment 1

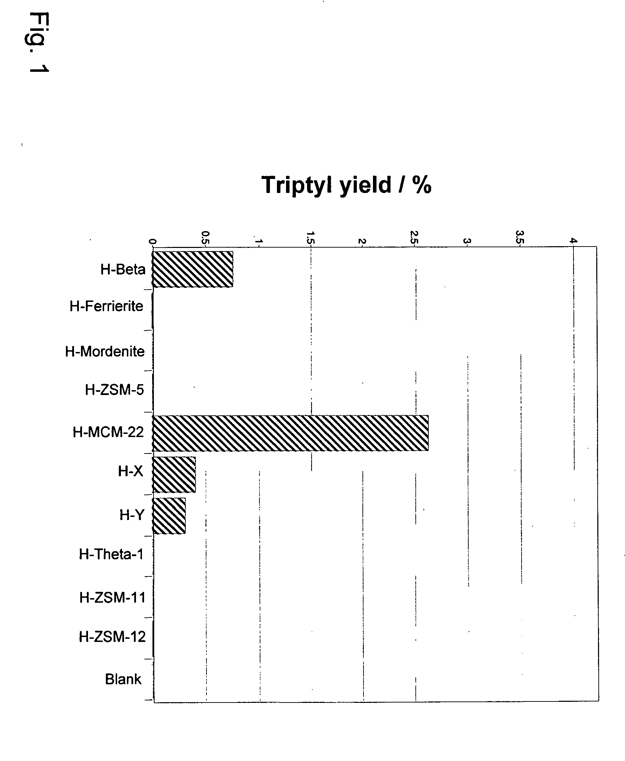

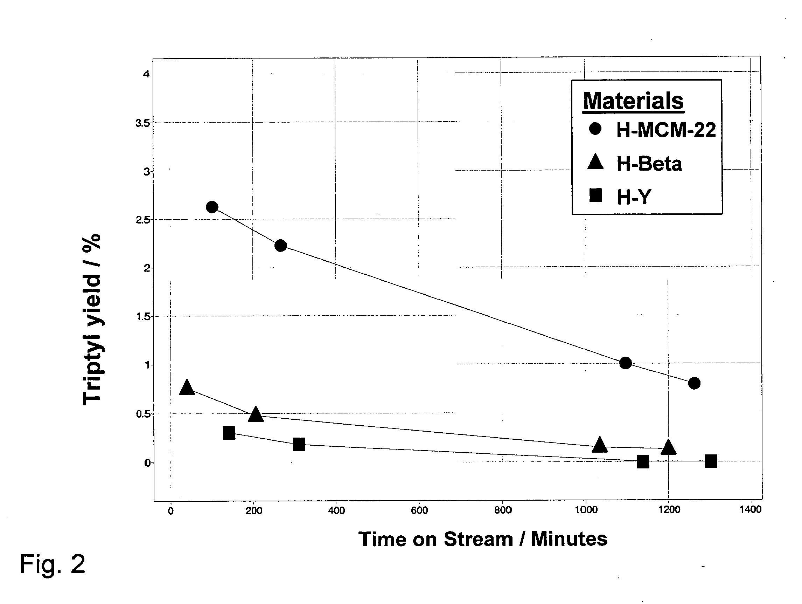

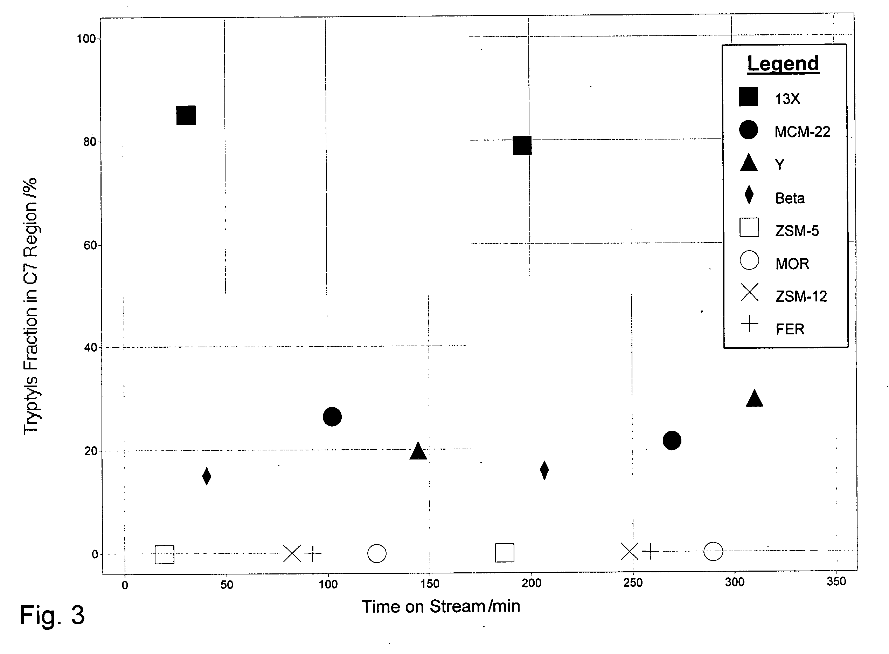

A mixture of methanol and n-propanol (10:1 1 molar ratio), diluted in nitrogen at a nitrogen : (methanol + n-propanol) molar ratio of 4:1, was fed over the catalyst at a pressure of 1 bara, and a temperature of 275°C, and a total GHSV of 1000 $h^{-1}$. The zeolites studied were X, Y, VUSY, MCM-22, Beta, ZSM-5, Mordenite, ZSM-12, Theta-1 and Ferrierite in the fully protonated form. Zeolites X, Y, Beta and MCM-22 resulted in the formation of triptyl compounds, giving triptyl fractions of 15.2% or more. The results are summarised in Table 1. Comparison of triptyl yields with the different catalysts is shown in Figure 1. The activity with time is shown for three of the zeolites in Figure 2, and plots of the triptyl fractions calculated for all the zeolites are illustrated in Figure 3.

Experiment 2

Experiment 1 was repeated, except the temperature was 350°C. The same four zeolites and VUSY were active for triptyl formation, giving triptyl fractions of 7% or more.

Experiment 3

Experiment 1 was repeated except the temperature was 200°C. Zeolites Beta, MCM-22 and Y were active for triptyl formation, giving triptyl fractions of 19.3% or more.

Table 1: Triptyl activity of various protonated zeolites using a nitrogen-diluted methanol/n-propanol feedstock at 275°C and a GHSV of 1000 h$^{-1}$.

| Material | Zeolite Type[a] | Accessible 12-Ring | All pores have 10-Ring or less. | Channel Structure | Triptyl Activity |
|---|---|---|---|---|---|
| H-ZSM-5 | MFI | no | Yes | 3D | No |
| H-X | FAU | Yes | no | 3D | **Yes[b]** |
| H-Beta | BEA | Yes | no | 3D | **Yes[b]** |
| H-ZSM-12 | MTW | Yes | no | 1D | No |
| H-MCM-22 | MWW | Yes | Yes | 2D | **Yes[b]** |
| H-Theta-1 | TON | no | Yes | 1D | No |
| H-Ferrierite | FER | no | Yes | 2D | No |
| H-Mordenite | MOR | Yes | no | 1D | No |
| H-Y | FAU | Yes | no | 3D | **Yes[b]** |
| [a] According to the "Atlas of Zeolite Structure Types" published by the International Zeolite Association. [b] "Yes" if triptyl yield was greater than 0.05% vs total carbon in the reaction composition. | | | | | |

Experiment 4

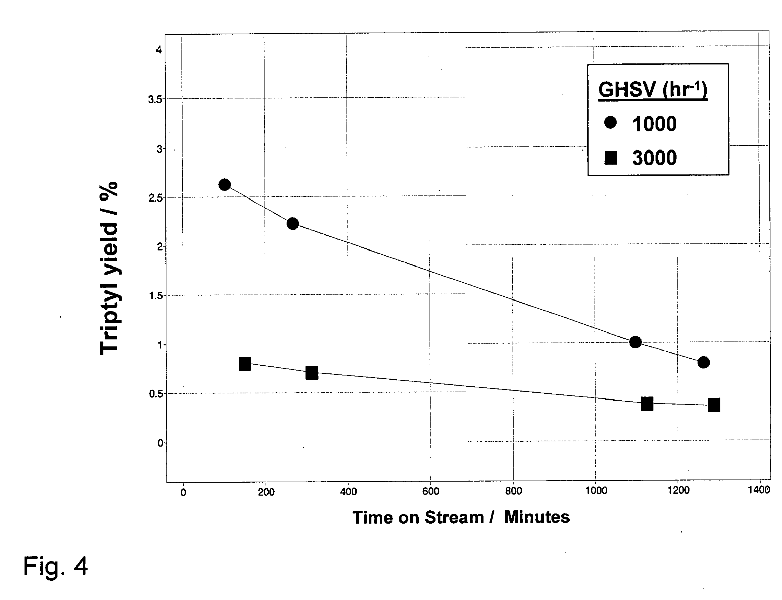

Experiment 1 was repeated, except the GHSV was increased to 3000 h$^{-1}$. The same catalysts were active giving triptyl fractions of 15.1 % or more, except for Zeolite X which did not exhibit triptyl activity. Results for MCM-22 are illustrated in Figure 4.

Experiment 5

Experiment 1 was repeated except that the GHSV was increased to 2000 h$^{-1}$, and only the following zeolites in their protonated form were tested; X, Y, Beta, MCM-22, Ferrierite and ZSM-12. The same four zeolites as Experiment 1 exhibited triptyl activity, giving triptyl fractions of 25.7% or more.

Experiment 6

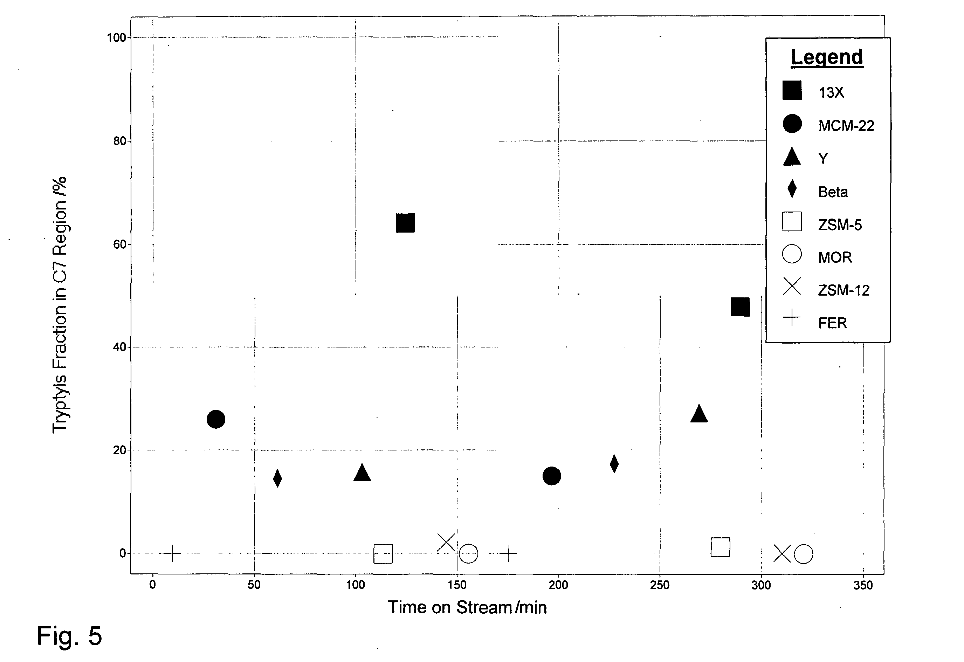

The catalysts listed in table 1 were tested for triptyl activity using a feedstock of dimethylether (DME) and propanol, in a 5 : 1 molar ratio, diluted in nitrogen in a nitrogen : (DME + propanol) molar ratio of 4 : 1, at a GHSV of 2000 h$^{-1}$ and a temperature of 275°C. The same catalysts as Experiment 1 showed activity towards triptyl formation. The triptyl fractions are shown in Figure 1, the active zeolites all providing triptyl fractions of 14.6% or more. Plots of the triptyl fractions obtained are shown in Figure 5.

Experiment 7

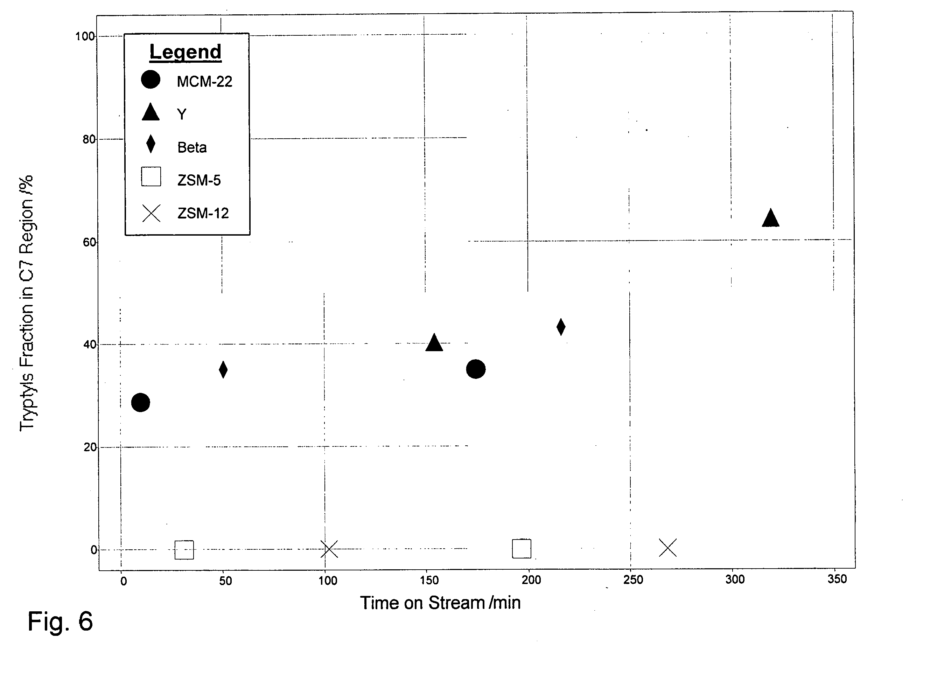

Protonated forms of zeolites X, Y, MCM-22, Beta, ZSM-5 and ZSM-12 were studied in the presence of a methanol feedstock, diluted in nitrogen at a nitrogen : methanol molar ratio of 4 : 1. Temperature was 275°C, and the GHSV was 2000 h$^{-1}$. Zeolites Y, MCM-22 and Beta showed triptyl activity, the triptyl fractions being 29% or more as illustrated in Figure 6.

Experiment 8

Protonated forms of zeolites X, Y, MCM-22, Beta, ZSM-5 and ZSM-12 were studied in the presence of a feedstock comprising methanol, ethanol, n-propanol and n-butanol in a molar ratio of, respectively, 10 : 1.04 : 0.23 : 0.06, diluted in nitrogen at a nitrogen : total alcohols molar ratio of 4 : 1. Temperature was 275°C, and the GHSV was 2000 h[-1]. Zeolites Y, MCM-22 and Beta showed triptyl activity, the triptyl fractions being 25.4% or more.

Experiment 9

Experiment 1 was repeated, except the GHSV was 2000 h[-1], and non-framework metal-loaded zeolites were tested. Ag, Ba, Bi, Co, Cu, Cs, In and Zn-loaded analogues of zeolites MCM-22, Beta, Y, X, Theta-1, H-ZSM-5, H-ZSM-12 and H-Theta-1 were evaluated. For each zeolite, metal-loaded materials were prepared having 1, 2.5 and 10mol% of the zeolite T-elements (in all cases, the only T-atom was Al). Li-exchanged forms of the above zeolites (except for Theta-1) were also prepared with loadings of 1 and 10mol% of aluminium (the T-element). Additionally, 1 and 10mol% Li-forms of Ferrierite were prepared. Results are summarised in Table 3. All metal-loaded zeolites produced triptyl in the case of MCM-22, Beta and zeolite Y, giving triptyl fractions of 5.5% or more. For zeolite X, only metals at greater than 1mol% loadings resulted in triptyl formation, these being Cu, Ag, In and Co. At greater than 2.5mol% loading, the Zn salt also formed triptyl. Where triptyl was formed, the triptyl fractions of metal-loaded Zeolite X were 44.3% or more.

Table 3 : Triptyl activity for nitrogen-diluted methanol/propanol feedstock at 275°C and 2000 GHSV[-1]

| Metal | Loading[a] | Zeolite | | | | | | |
|---|---|---|---|---|---|---|---|---|
| | | Beta | Y | MCM-22 | X | ZSM-5 | ZSM-12 | Theta-1 |
| Ag | 1% | **Yes** | **Yes** | **Yes** | no | no | no | no |
| | 2.5% | **Yes** | **Yes** | **Yes** | **Yes** | no | no | no |
| | 10% | **Yes** | **Yes** | **Yes** | **Yes** | no | no | no |
| Ba | 1% | **Yes** | **Yes** | **Yes** | no | no | no | no |
| | 2.5% | **Yes** | **Yes** | **Yes** | no | no | no | no |
| | 10% | **Yes** | **Yes** | **Yes** | no | no | no | no |
| Bi | 1% | **Yes** | **Yes** | **Yes** | no | no | no | no |
| | 2.5% | **Yes** | **Yes** | **Yes** | no | no | no | no |
| | 10% | **Yes** | **Yes** | **Yes** | no | no | no | no |
| Co | 1% | **Yes** | **Yes** | **Yes** | no | no | no | no |
| | 2.5% | **Yes** | **Yes** | **Yes** | **Yes** | no | no | no |
| | 10% | **Yes** | **Yes** | **Yes** | **Yes** | no | no | no |
| Cu | 1% | **Yes** | **Yes** | **Yes** | no | no | no | no |
| | 2.5% | **Yes** | **Yes** | **Yes** | Yes | no | no | no |
| | 10% | **Yes** | **Yes** | **Yes** | Yes | no | no | no |
| Cs | 1% | **Yes** | **Yes** | **Yes** | no | no | no | no |
| | 2.5% | **Yes** | **Yes** | **Yes** | no | no | no | no |
| | 10% | **Yes** | **Yes** | **Yes** | no | no | no | no |
| In | 1% | **Yes** | **Yes** | **Yes** | no | no | no | no |
| | 2.5% | **Yes** | **Yes** | **Yes** | **Yes** | no | no | no |
| | 10% | **Yes** | **Yes** | **Yes** | **Yes** | no | no | no |
| Li[b] | 1% | **Yes** | **Yes** | **Yes** | no | no | no | no[c] |
| | 2.5% | - | - | - | - | - | - | - |
| | 10% | **Yes** | **Yes** | **Yes** | Yes | no | no | no[c] |
| Zn | 1% | **Yes** | **Yes** | **Yes** | no | no | no | no |
| | 2.5% | **Yes** | **Yes** | **Yes** | no | no | no | no |

(continued)

| Metal | Loadinga | Zeolite | | | | | | |
|---|---|---|---|---|---|---|---|---|
| | | Beta | Y | MCM-22 | X | ZSM-5 | ZSM-12 | Theta-1 |
| | 10% | **Yes** | **Yes** | **Yes** | **Yes** | no | no | no |
| a Expressed as a percentage of aluminium (the only T-element) in the zeolite. | | | | | | | | |
| b Experiments carried out at a GHSV of 3000 h-1. | | | | | | | | |
| c The zeolite for these experiments was Li-exchanged Ferrierite, not Theta-1. | | | | | | | | |

Experiment 10

In this Experiment, the catalyst bed comprised an upstream bed of a commercial $Cu/ZnO/Al_2O_3$ methanol synthesis catalyst from Engelhard mixed with gamma alumina, and a downstream bed of protonated zeolite selected from Beta, ZSM-5, MCM-22, X and Y. The zeolite catalysts were pre-treated by calcining under air at 400°C. When the two catalysts were loaded into the reactor, they were first pre-treated in a flow of 20% hydrogen in nitrogen at 200°. A mixture of hydrogen and carbon monoxide, at a $H_2$ : CO molar ratio of 5:1, was then passed over the combined catalyst at a GHSV of 2000 h-1 (with respect to the entire catalyst bed). Reaction pressure was 50 bara, and the reaction temperature was 275°C. Zeolites Beta, MCM-22 and Y were active for triptyl formation, giving triptyl fractions of 34.7% or more. Table 4 summarises the results.

Table 4 : Triptyl activity using $CO/H_2$ feedstock.

| Zeolite | Time on Stream (min) | Triptyl Yield (%)a | Triptyl Fraction (%) |
|---|---|---|---|
| MCM-22 | 123.5 | 0.4 | 53 |
| Y | 112.5 | 0.3 | 36 |
| Beta | 92.5 | 0.3 | 48 |
| ZSM-5 | 133.5 | 0.0 | 0 |
| a Versus CO in the feedstock. | | | |

Experiment 11

Zeolites tested were ZSM-5 and MCM-22, both in their fully protonated form. The Experiment was related to Experiment 5, except that a larger reactor and catalyst bed were used. Results and reaction conditions for this Experiment are shown in Table 5.

3ml of the zeolite catalyst was physically mixed with 3ml of Grace 57 silica to achieve a 1:1 volume dilution of the zeolite catalyst. The mixture was then loaded into a quartz reactor of 8.8mm internal diameter. A pre-bed of 6ml of silicon carbide was also loaded inside the tube upstream of the zeolite catalyst to ensure full vaporisation and good mixing of the reaction composition before contact with the zeolite catalyst. The reactor was used in a single-pass, down-flow configuration with the feedstock being introduced at the top of the reactor tube.

Nitrogen was passed through the reactor tube and over the catalyst bed at a constant flow rate. The reactor tube was heated using a Carbolite tube furnace and was set to heat to the required temperature at a heating rate of 3.0°C/min. When the reaction temperature was reached, a liquid feed mixture of methanol and n-propanol at the desired molar ratio was fed to the top of the reactor tube using a syringe pump set at constant flow rate to achieve the desired nitrogen to alcohols (methanol + n-propanol) molar ratio. The resulting gaseous product stream was passed through a condenser vessel maintained at a temperature of 5°C. The liquid collected was weighed at the end of the experiment for assessing the mass balance. The uncondensed portion of the product stream was sampled periodically, and the samples analysed by GC to determine the composition. The volume of the uncondensed product stream was continuously measured. GC analysis was conducted using a Chrompak CP9000 GC, fitted with a single FID detector and a single boiling point column (CP SIL 8; 50m, 0.32mm, 1.2m). The temperature programme involved maintaining an initial column temperature of 50°C for 10 minutes, followed by an increase to 120°C at a heating rate of 6°C/min. Total analysis time was approximately 22 minutes with triptyls products eluting at ca. 11 minutes.

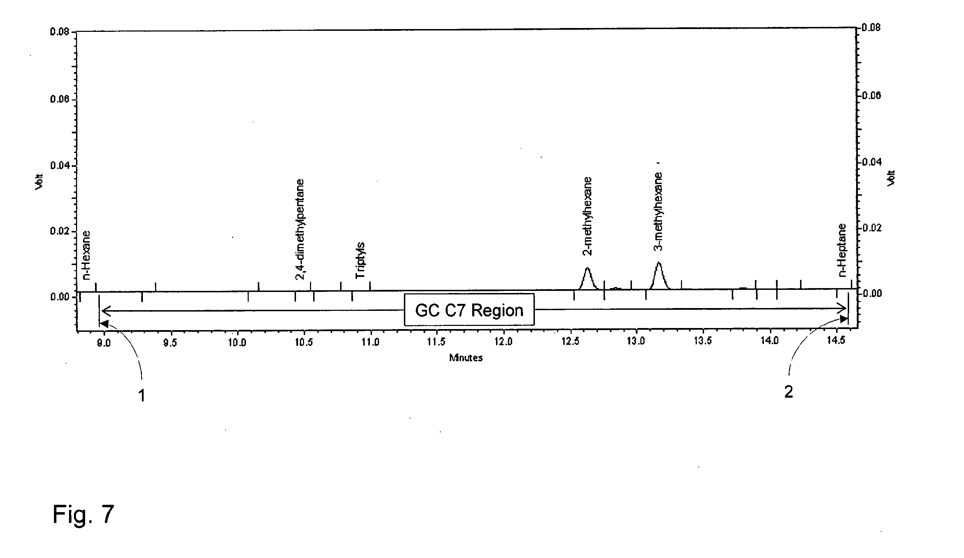

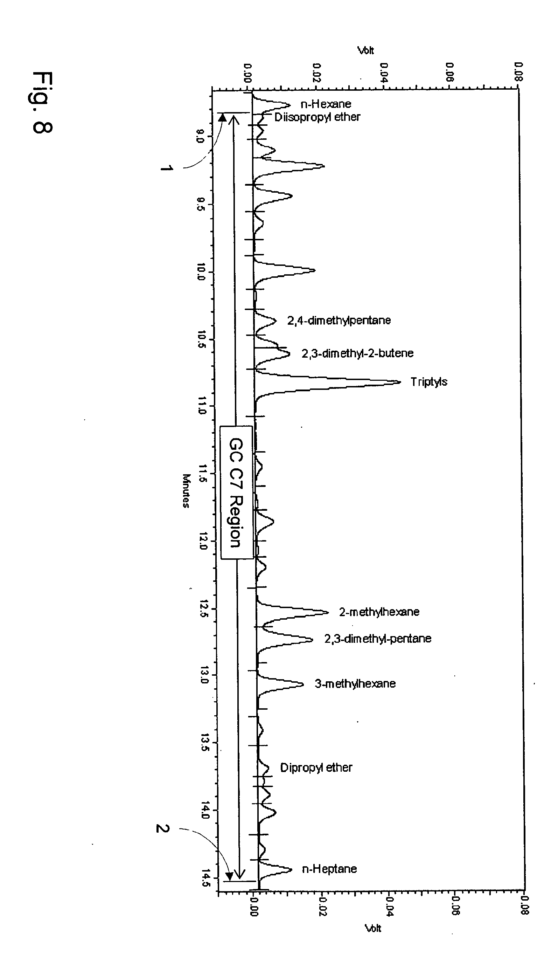

Representative GC traces are shown in Figures 7 and 8, which also highlight the region of the GC trace used to define the $C_7$ hydrocarbon region, and which is used in calculating the triptyl fraction, that is the region with a retention time greater than n-hexane, indicated by 1, up to and including n-heptane, indicated by 2.

Table 5 : Performance of ZSM-5 and MCM-22 catalysts for triptyl formation using nitrogen-diluted methanol/n-propanol feedstock.

| | ZSM-5 | ZSM-5 | MCM-22 | MCM-22 |
|---|---|---|---|---|
| Time on stream (min) | 11 | 256 | 25 | 258 |
| Temperature (°C) | 250 | 250 | 275 | 275 |
| GHSV (h$^{-1}$) | 2000 | 2000 | 2000 | 2000 |
| $C_1/C_3$ alcohol mole Ratio | 20 : 1 | 20 : 1 | 10 : 1 | 10 : 1 |
| $N_2$:alcohol mole ratio | 9:1 | 9 : 1 | 4 : 1 | 4 : 1 |
| Triptyls fraction (%) | 0.7 | 0.8 | 25.6 | 11.8 |
| Triptyls yield (%) | <0.02 | <0.02 | 1.5 | 0.8 |

## Claims

1. A process for the production of triptane and/or triptene from methanol and/or one or more derivatives thereof and optionally one or more further alcohols and/or derivatives thereof, which process comprises contacting a reaction composition comprising methanol and/or one or more derivatives thereof, and optionally one or more further alcohols and/or derivatives thereof, with a catalyst having Brønsted acidity to produce triptane and/or triptene at a combined yield of greater than 0.05% at a temperature in the range of from 150 to 400°C, in which all the carbon atoms of the triptane and/or triptene are derived from the methanol and/or one or more derivatives thereof and the optional one or more further alcohols and/or derivatives thereof, **characterised in that** the catalyst is selected from one or more of:

    i. zeolites having frameworks comprising silicon and aluminium atoms at a Si:Al mole ratio of greater than 2, which zeolites also have a channel structure comprising a ring size of 12 or more non-oxygen atoms in 2 or 3 dimensions; and
    ii. zeolites having frameworks comprising silicon and aluminium atoms, and which have a framework ring comprising 12 or more non-oxygen-atoms accessible on the external surface of the zeolite and a pore structure in which all of the channels have a ring-size of less than 12 non-oxygen atoms.

2. A process as claimed in claim 1, in which the zeolite catalyst is selected from (i) and has the FAU or BEA structure.

3. A process as claimed in claim 2, in which the zeolite catalyst is zeolite Y or zeolite Beta.

4. A process as claimed in claim 1, in which the zeolite catalyst is selected from (ii) and has the MWW structure.

5. A process as claimed in claim 4, in which the zeolite catalyst is MCM-22 or ITQ-2.

6. A process as claimed in any one of claims 1 to 5, in which the zeolite catalyst comprises non-framework metal ions.

7. A process as claimed in claim 6, in which the non-framework metal ions are selected from one or more of Ag, Ba, Bi, Ca, Ce, Cu, Co, Cs, In, La, Li, Rh, Pd, Pt and Zn.

8. A process as claimed in claim 7, in which the non-framework metal ions are selected from one or more of Ag, Co, Cu and In.

9. A process as claimed in any one of claims 1 to 8, in which the zeolite catalyst is an aluminosilicate zeolite.

10. A process as claimed in any one of claims 1 to 9, in which the zeolite comprises up to 10mol% of framework heteroatoms selected from one or more of phosphorus, gallium, titanium, germanium, vanadium, iron and cobalt.

**11.** A process as claimed in any one of claims 1 to 10, in which one or more further alcohols and/or derivatives thereof selected from $C_2$ to $C_4$ alcohols and/or derivatives thereof are present in the reaction composition.

**12.** A process as claimed in claim 11, in which at least one of the further alcohols and/or derivatives thereof is a $C_3$ alcohol and/or derivative thereof.

**13.** A process for the production of triptane and/or triptene from methanol and/or one or more derivatives thereof, a $C_3$ alcohol and/or one or more derivatives thereof, and optionally one or more further alcohols and/or derivatives thereof, which process comprises contacting a reaction composition comprising methanol and/or one or more derivatives thereof, the $C_3$ alcohol and/or one or more derivatives thereof and optionally one or more further alcohols and/or derivatives thereof with a zeolite catalyst having Brønsted acidity to produce triptane and/or triptene at a combined yield of greater than 0.05% at a temperature in the range of from greater than 200 and up to 400°C, in which all the carbon atoms of the triptane and/or triptene are derived from the methanol and/or one or more derivatives thereof, the $C_3$ alcohol and/or one or more derivatives thereof, and optionally the one or more further alcohols and/or derivatives thereof, **characterised in that** the zeolite catalyst is zeolite X, and the mole ratio of $C_3$ alcohol : methanol present in the reaction composition, and/or derivable from the one or more derivatives thereof present in the reaction composition, is greater than 0.23 : 10.

**14.** A process as claimed in claim 13, in which the zeolite X comprises one or more non-framework elements selected from Ag, Li, Co, Cu and In at a loading of greater than 1mol% of the T-element content of the Zeolite X.

**15.** A process as claimed in claim 13 or claim 14, in which one or more further alcohols and/or derivatives thereof selected from $C_2$ and $C_4$ alcohols and/or derivatives thereof are present in the reaction composition.

**16.** A process as claimed in any one of claims 1 to 15, in which the GHSV is in the range of 500 to 5000 h$^{-1}$.

**17.** A process as claimed in claim 16, in which the GHSV is less than 3000 h$^{-1}$.

**18.** A process as claimed in any one of claims 1 to 17, in which the reaction composition comprises methanol and/or dimethyl ether.

**19.** A process as claimed in any one of claims 1 to 18, in which the methanol and/or one or more derivatives thereof, and optionally one or more further alcohols and/or derivatives thereof, are produced by contacting a mixture of carbon monoxide and hydrogen with an alcohols synthesis catalyst active for the production of methanol and/or derivatives thereof, and optionally further alcohols and/or derivatives thereof.

**20.** A process as claimed in claim 19, in which the alcohols synthesis catalyst and the zeolite catalyst are in the same reactor.

**21.** A process as claimed in claim 20, in which the alcohols synthesis catalyst and the zeolite catalyst are in separate layers.

**22.** A process as claimed in any one of claims 19 to 21, in which the process operates at a temperature in the range of from 240°C to below 310°C, and a pressure of at least 10 bara (1 MPa).

**23.** A process as claimed in claim 19, in which the alcohols synthesis catalyst is in a first reactor, and the zeolite catalyst is in a second reactor, wherein carbon monoxide and hydrogen are fed to the first reactor to produce a product composition comprising methanol and/or derivatives thereof, and optionally further alcohols and/or derivatives thereof, which product composition is then fed to the second reactor in which the process as claimed in any one of claims 1 to 18 takes place.

**24.** A process as claimed in claim 23, in which product composition from the first reactor is treated to remove unreacted carbon monoxide and hydrogen before being fed to the second reactor.

**25.** A process as claimed in claim 23 or claim 24, in which the first reactor operates at a temperature in the range of from (150 to 400°C) and a pressure in the range of from 10 to 150 bara (1 to 15 MPa).

**26.** A process as claimed in any one of claims 19 to 25, in which the $H_2$ : CO mole ratio is in the range of from 1:1 to 6:1.

**27.** A process as claimed in any one of claims 19 to 26, in which the alcohol synthesis catalyst is $Cu/Zno/Al_2O_3$ or CoMoS.

**28.** A process as claimed in any one of claims 1 to 27, in which the triptyl fraction is greater than 5%.

Fig. 1

Fig. 2

Fig. 3

Tryptyls Fraction in C7 Region /%

**Fig. 4**

Triptyl yield / %

Time on Stream / Minutes

GHSV (hr⁻¹)
● 1000
■ 3000

Fig. 5

Tryptyls Fraction in C7 Region /%

Fig. 6

Time on Stream /min

Tryptyls Fraction in C7 Region /%

Legend

MCM-22 ●
Y ▼
Beta ◆
ZSM-5 □
ZSM-12 ✕

19

Fig. 7

Fig. 8

**European Patent Office**

**EUROPEAN SEARCH REPORT**

Application Number

EP 07 25 4486

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JP 62 121787 A (MOBIL OIL CORP) 3 June 1987 (1987-06-03) * abstract * | 1-28 | INV. C07C1/20 C07C9/16 C07C11/02 B01J29/06 |
| A | EP 0 090 232 A (BASF AG [DE]) 5 October 1983 (1983-10-05) * page 2, line 32 - line 39 * table on page 5, example 4 | 1-28 | |
| A | WO 2005/023733 A (BP OIL INT [GB]; KAY RICHARD DANIEL [GB]; MORRIS GEORGE ERNEST [GB]; S) 17 March 2005 (2005-03-17) * the whole document * | 1-28 | |
| A | WO 97/45198 A (EXXON CHEMICAL PATENTS INC [US]) 4 December 1997 (1997-12-04) page 25 (U)* example 6 * | 1-28 | |
| | | | TECHNICAL FIELDS SEARCHED (IPC) |
| | | | C07C B01J |

The present search report has been drawn up for all claims

| Place of search | Date of completion of the search | Examiner |
|---|---|---|
| Munich | 23 April 2008 | Breimaier, Waltraud |

EPO FORM 1503 03.82 (P04C01)

**ANNEX TO THE EUROPEAN SEARCH REPORT**
**ON EUROPEAN PATENT APPLICATION NO.**                    EP 07 25 4486

This annex lists the patent family members relating to the patent documents cited in the above-mentioned European search report.
The members are as contained in the European Patent Office EDP file on
The European Patent Office is in no way liable for these particulars which are merely given for the purpose of information.

23-04-2008

| Patent document cited in search report | | Publication date | Patent family member(s) | | Publication date |
|---|---|---|---|---|---|
| JP 62121787 | A | 03-06-1987 | ZA | 8607243 A | 27-04-1988 |
| EP 0090232 | A | 05-10-1983 | DE | 3210756 A1 | 29-09-1983 |
| WO 2005023733 | A | 17-03-2005 | AR | 046681 A1 | 21-12-2005 |
| | | | AT | 349408 T | 15-01-2007 |
| | | | AU | 2004270443 A1 | 17-03-2005 |
| | | | BR | PI0414092 A | 31-10-2006 |
| | | | CA | 2536798 A1 | 17-03-2005 |
| | | | CN | 1845887 A | 11-10-2006 |
| | | | DE | 602004003964 T2 | 18-10-2007 |
| | | | EP | 1660417 A2 | 31-05-2006 |
| | | | ES | 2279440 T3 | 16-08-2007 |
| | | | JP | 2007504206 T | 01-03-2007 |
| | | | US | 2007004955 A1 | 04-01-2007 |
| WO 9745198 | A | 04-12-1997 | AU | 737308 B2 | 16-08-2001 |
| | | | AU | 3297697 A | 05-01-1998 |
| | | | BR | 9709398 A | 10-08-1999 |
| | | | CN | 1223603 A | 21-07-1999 |
| | | | DE | 69729797 D1 | 12-08-2004 |
| | | | DE | 69729797 T2 | 02-12-2004 |
| | | | EA | 2220 B1 | 28-02-2002 |
| | | | EP | 0909216 A1 | 21-04-1999 |
| | | | ES | 2221054 T3 | 16-12-2004 |
| | | | JP | 2000511106 T | 29-08-2000 |
| | | | KR | 20000016115 A | 25-03-2000 |
| | | | TW | 380121 B | 21-01-2000 |
| | | | ZA | 9704712 A | 17-11-1998 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9822556 A
- WO 9949003 A
- GB 1547955 A
- US 2492984 A
- US 3969427 A
- US 4059646 A
- US 4059647 A
- US 4249031 A
- WO 0270440 A
- US 4151214 A
- US 3894102 A
- US 3894107 A
- US 3308069 A
- US 3415736 A
- US 3972983 A
- US 4992615 A
- WO 0121562 A